Europäisches Patentamt

European Patent Office

Office européen des brevets

(11) Numéro de publication : **0 392 943 B1**

(12) **FASCICULE DE BREVET EUROPEEN**

(45) Date de publication du fascicule du brevet :
**08.12.93 Bulletin 93/49**

(51) Int. Cl.$^5$ : **G01N 15/02, D21F 1/00**

(21) Numéro de dépôt : **90420180.3**

(22) Date de dépôt : **11.04.90**

(54) **Dispositif de détermination de caractéristiques de particules en suspension dans un liquide.**

(30) Priorité : **12.04.89 FR 8905162**

(43) Date de publication de la demande :
**17.10.90 Bulletin 90/42**

(45) Mention de la délivrance du brevet :
**08.12.93 Bulletin 93/49**

(84) Etats contractants désignés :
**AT BE DE ES FR GB IT SE**

(56) Documents cités :
**WO-A-88/02855**
**US-A- 4 692 210**
**ADVANCES IN INSTRUMENTATION, vol. 30,**
**part 2, 1975, pages 668(1-12), ISA AC, Chicago,**
**US; T. BAY et al.: "Continuous particle size**
**analysis and grinding control"**
**TAPPI, vol. 53, no. 7, juillet 1970, pages**
**1240-1247; G. OLGARD: "Fractionation of fiber**
**suspensions by liquid column flow"**

(73) Titulaire : **ASSOCIATION DE GESTION DE**
**L'ECOLE FRANCAISE DE PAPETERIE ET DE**
**L'IMPRIMERIE**
**154, Boulevard Haussmann**
**F-75008 Paris (FR)**
Titulaire : **Silvy, Jacques**
**6, Rue Hector Berlioz**
**F-38000 Grenoble (FR)**
Titulaire : **Pascal, René**
**7, Allée des Eyminées**
**F-38240 Meylan (FR)**

(72) Inventeur : **Silvy, Jacques**
**6, Rue Hector Berlioz**
**F-38000 Grenoble (FR)**
Inventeur : **Pascal, René**
**7, Allée des Eyminées**
**F-38240 Meylan (FR)**

(74) Mandataire : **de Beaumont, Michel**
**1bis, rue Champollion**
**F-38000 Grenoble (FR)**

## Description

La présente invention concerne un dispositif de détermination des caractéristiques de particules en suspension dans un liquide. La présente invention s'applique plus particulièrement à la caractérisation des constituants de pâtes à papier au cours de la fabrication du papier. Elle permet notamment de mesurer des paramètres tels que longueur, largeur, courbure, et proportion entre les éléments organiques et les éléments minéraux.

Les suspensions comme les pâtes à papier comprennent des éléments organiques fibreux et des éléments minéraux de formes, de dimensions et en proportions extrêmement variées qu'il importe de connaître car les propriétés du produit fini dépendent en grande partie de ces paramètres. Les particules comprennent des fibres dont la plus grande dimension varie entre quelques fractions de millimètre et plus d'un centimètre qui sont en suspension dans un liquide, couramment de l'eau.

On connaît dans l'art antérieur de nombreux procédés de détermination de caractéristiques de particules en suspension telles que des fibres et des charges minérales contenues dans une pâte à papier.

On a proposé (voir par exemple US-A-4 692 210) des appareils dans lesquels les particules en suspension dans un liquide sont amenées à passer par une veine transparente qui est éclairée, par exemple par une source de lumière cohérente, ce qui permet d'effectuer les diverses mesures suivantes :
- mesure de longueur de fibres par formation de l'image des fibres sur des lignes de photodétecteurs et comptage du nombre de photodétecteurs impressionnés,
- mesure d'une dimension caractéristique de leur diamètre par analyse de figures de diffraction et de diffusion, et
- mesure de la proportion fibre cellulosique/charge minérale par la mesure de la dépolarisation du flux direct d'une source de lumière.

Dans le cas de la mesure de longueur, ce type d'appareils présente l'inconvénient d'exiger l'utilisation d'une veine de mesure optique constituée d'un capillaire de petite section de façon à limiter strictement le nombre de fibres qui la parcourt à un instant donné pour pouvoir ainsi effectuer des images de fibres individuelles et des mesures de longueur de fibres. En effet, si un grand nombre de fibres arrive simultanément, leurs images ne peuvent être séparées et la mesure de longueur ne peut être effectuée. L'inconvénient de l'utilisation d'un capillaire de petite section est, outre l'augmentation du temps de mesure, le fait qu'en pratique un tel capillaire est susceptible d'être bouché par une accumulation de particules ou l'arrivée d'une particule de section plus importante. Il en résulte que les procédés de mesure à veine optique susmentionnés ne sont pas utilisés dans le cadre de fabrications industrielles mais uniquement en laboratoire, par prélèvement d'échantillons et examen séparé de ceux-ci.

Indépendamment des appareils de mesure de paramètres caractéristiques de la morphologie de fibres ou autres éléments en suspension dans un liquide, on connaît dans l'art antérieur des appareils de fractionnement de particules, utilisés en papeterie, qui permettent de classer les constituants d'une pâte à papier en fonction de la longueur des fibres. Un exemple d'un tel appareil est décrit dans un article de Gunnar OLGARD paru dans TAPPI, Vol. 53, N°7, juillet 1970, PP 1240-1270.

Un tel appareil de fractionnement, ou fractionneur, consiste en un tuyau dans lequel circule un liquide (éluant) entre deux bulles d'air et dans lequel on injecte des particules déjà en suspension. Les particules sont alors concentrées en suspension et classées. En papeterie, l'éluant sera généralement de l'eau. En envoyant la sortie d'un tel appareil de fractionnement hydrodynamique successivement dans des récipients différents, par exemple placés sur un carrousel, chacun des récipients se remplit d'éluant contenant des fibres dans une gamme de longueurs donnée.

Un fractionneur n'est pas un appareil de mesure mais un appareil de tri.

Un objet de la présente invention est de prévoir un appareil de mesure de la quantité de particules fractionnées par intervalle de taille ainsi que de paramètres caractéristiques de fibres ou autres composants en suspension dans un liquide, cet appareil présentant l'avantage de pouvoir être utilisé dans un environnement industriel et de permettre un suivi périodique, pratiquement en temps réel, d'une pâte à papier en suspension circulant dans des conduites de traitement.

La présente invention se base sur l'observation que, parmi les paramètres qui peuvent être mesurés par passage dans une veine de mesure optique, pour la mesure de certains paramètres seulement, on cherche à faire une analyse individuelle sur une fibre (mesure de longueur des fibres pour laquelle on cherche à faire l'image individuelle de chaque fibre), ce qui impose l'utilisation d'un capillaire de petite dimension. Par contre, pour les autres paramètres, on analyse les caractéristiques globales de la lumière diffusée, diffractée, et/ou dépolarisée et on obtient des résultats statistiques qui ne nécessitent pas d'analyse sur une fibre ou autre élément pris individuellement.

Ainsi, la présente invention prévoit d'associer dans un même appareil un fractionneur hydrodynamique et une veine de mesure optique recevant directement la sortie du fractionneur. Ce dernier est utilisé pour fournir sous forme diluée et classée par ordre de taille des échantillons de fibres qui y sont introduits. Les instants d'arrivée des fibres dans la veine de mesure par rapport à l'instant d'injection dans le fractionneur donnent, en relation avec un calibrage, une mesure de la longueur des fibres tandis que la quantité de ces fibres ainsi que les autres paramètres associés sont détectés par des capteurs optiques et autres organes de traitement associés à la veine de mesure optique.

On peut alors analyser un grand nombre de fibres circulant simultanément et il n'est plus nécessaire de prévoir un capillaire de très petite dimension pour la veine de mesure optique. L'appareil de mesure selon la présente invention peut donc fonctionner à haut débit et sans risque d'encrassement.

Plus particulièrement, la présente invention prévoit un dispositif de détermination de caractéristiques de particules en suspension dans un liquide, comprenant des moyens pour injecter des échantillons de particules ; un fractionneur qui classe ces particules en fonction d'un premier paramètre et les fournit à sa sortie de façon ordonnée et déterminée temporellement en fonction de ce premier paramètre ; une veine de mesure optique recevant la sortie du fractionneur, cette veine de mesure optique comprenant des moyens pour envoyer un rayonnement électromagnétique cohérent polarisé à travers le flux de particules en suspension et pour recueillir la lumière diffractée, éventuellement dépolarisée, partiellement absorbée et diffusée dans diverses directions et des moyens de calcul des valeurs des intensités et de combinaisons de ces valeurs dans les diverses directions du rayonnement optique.

Selon un mode de réalisation de la présente invention, l'élément de fractionnement est un classeur hydrodynamique diluant la suspension effectuant un classement privilégiant la longueur des particules, cet élément étant constitué d'un tuyau dont la section est faible par rapport à sa longueur, des quantités données d'échantillon de particules en suspension dans un liquide étant injectées à des instants déterminés dans le tuyau dans lequel un éluant circule à débit controlé.

Selon un mode de réalisation de la présente invention, à la sortie de la veine de mesure optique, est prévu un carrousel dont le fonctionnement est synchronisé avec les périodes d'injection d'échantillon de particules en suspension pour recueillir des fractions successives du fluide contenant des particules classées selon le premier paramètre.

Selon un mode de réalisation de la présente invention, un automate programmable assure le séquencement entre l'injection des échantillons dans l'élément de fractionnement et les prises de mesure par des capteurs associés à la veine de mesure optique pour mesurer notamment à chaque instant correspondant à des particules de longueur donnée, leur quantité, leur largeur, leur courbure et la proportion entre les éléments minéraux et organiques.

Selon un mode de réalisation de la présente invention, les moyens pour recueillir la lumière comprennent des photodétecteurs disposés selon un cercle pour permettre une analyse sectorielle de l'intensité lumineuse diffractée et les moyens de calcul comprennent des moyens pour en déduire un facteur de forme des particules dans la veine de mesure.

Les particules se trouvant diluées et triées en fonction de leur longueur par passage dans l'appareil de fractionnement hydrodynamique, la sortie du dispositif selon l'invention est également classée en fonction de la longueur des particules. On peut donc orienter celle-ci vers des sorties différentes lors de périodes temporelles successives, pour permettre d'effectuer des mesures complémentaires sur des particules triées.

Ces objets, caractéristiques et avantages ainsi que d'autres de la présente invention seront exposés plus en détail dans la description suivante de modes de réalisation particuliers faite en relation avec les figures jointes parmi lesquelles :

la figure 1 représente, partiellement sous forme de blocs, l'agencement général d'un dispositif selon la présente invention ;

la figure 2 représente un exemple de préleveur automatique d'échantillon sur une conduite de pâte à papier ,

la figure 3 représente le schéma de principe de la veine de mesure et de ses dispositifs associés ,

la figure 4 représente un exemple de disposition d'éléments photosensibles dans le plan de l'image de diffraction ,

la figure 5 représente l'allure des variations d'intensité lumineuse mesurée par des détecteurs photosensibles permettant de caractériser des particules de différents diamètres ,

la figure 6 représente une courbe d'intensité lumineuse en fonction du temps au voisinage de l'axe optique du système de mesure ; et

la figure 7 représente une courbe de calibrage de longueurs de fibres en fonction du temps pour un appareil selon l'invention.

## STRUCTURE D'ENSEMBLE DE L'INVENTION

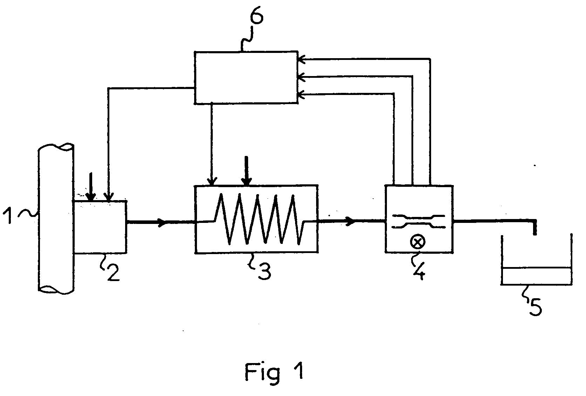

La figure 1 représente de façon schématique et sous forme de blocs l'organisation d'ensemble d'un dispositif de mesure des caractéristiques d'éléments dilués dans l'eau, tels que des fibres et des charges minérales, circulant dans une conduite 1 d'une papeterie. Un organe préleveur 2 permet d'effectuer un prélèvement d'échantillon de la pâte à papier circulant dans la conduite 1 à des instants souhaités puis de l'injecter dans un dispositif de fractionnement 3 constitué par exemple d'un tuyau de grande longueur alimenté en permanence en fluide. La sortie du fractionneur 3 est dirigée vers une veine de mesure optique 4 et, après mesure, le fluide est déversé dans un réservoir 5 et éventuellement récupéré. La synchronisation du système est assurée par un automate programmable 6 qui envoie des signaux appropriés aux diverses vannes et prélève à des instants choisis les sorties des capteurs associés au dispositif de mesure 4. Cet automate programmable peut comprendre un microprocesseur. Le fractionneur 3 envoie à la veine de mesure des particules de longueurs séquentiellement décroissantes et la veine de mesure 4 sert à mesurer la quantité de fibres dans les différentes séquences ainsi que les caractéristiques des fibres et charges minérales autres que leurs longueurs.

## PRÉLEVEUR

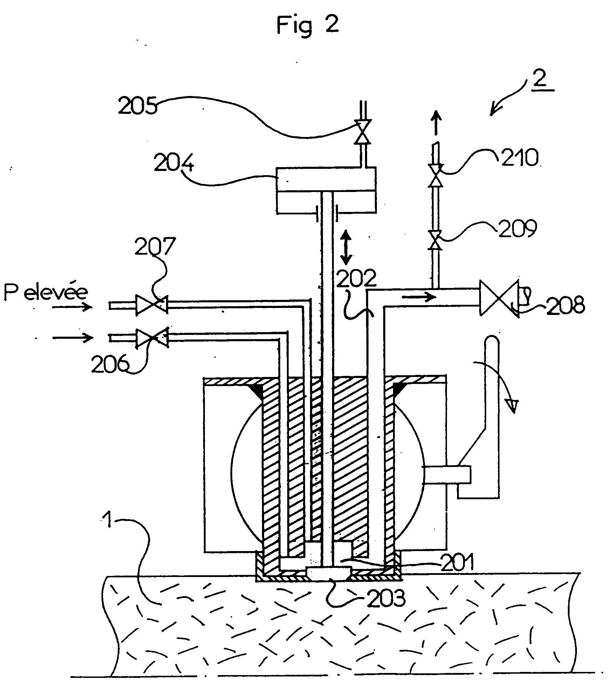

La figure 2 illustre un exemple de réalisation du préleveur 2. Celui-ci comprend une chambre 201 prolongée par une portion de conduite 202. La chambre 201 est susceptible de communiquer avec la conduite 1 par une soupape 203 commandée par un actionneur 204 piloté par un distributeur 205 lui-même commandé par l'automate programmable 6. Des arrivées d'eau vers la chambre 201 et la conduite 202 sont commandées par des vannes 206 et 207, la vanne 206 recevant de l'eau à pression normale et la vanne 207 de l'eau à haute pression, et notamment à une pression supérieure à celle régnant dans la conduite 1. La portion de conduite 202 communique par une vanne 208 avec l'entrée du fractionneur 3. Une vanne 209 et un limiteur de débit 210 permettent de purger la portion de conduite 202. Toutes les vannes précédemment énumérées agissent sous la commande de l'automate programmable 6.

Une opération de prélèvement suit les étapes indiquées dans le tableau 1 ci-dessous dans lequel la lettre F désigne une phase de fermeture d'une vanne et la lettre O une phase d'ouverture.

### TABLEAU 1

|  | 207 | 206 | 208 | 203 | 209 |
|---|---|---|---|---|---|
| Phase 1 | F | O | O | F | F |
| Phase 2 | F | F | F | F | F |
| Phase 3 | F | F | F | O | O |
| Phase 4 | O | F | F | F | F |
| Phase 1 | F | O | O | F | F |

La succession des phases de fonctionnement pour effectuer un prélèvement est la suivante :

Phase 1. Les vannes 206 et 208 sont ouvertes, les autres vannes étant fermées, de l'eau circule dans la chambre 201 et la conduite 202 pour sortir vers le fractionneur 3. C'est la phase de plus longue durée pendant laquelle un échantillon préalablement prélevé circule dans le fractionneur.

Phase 2. Pendant une très courte durée, toutes les vannes sont fermées.

Phase 3. Les vannes d'entrée d'eau pure 206 et 207 et la vanne de sortie 208 sont fermées, la soupape 203 est ouverte ainsi que la vanne 209 et la chambre 201 et la portion de conduite 202 se remplissent de la pâte en suspension en provenance de la conduite 1 à un débit constant déterminé par le limiteur 210 pour admettre un volume donné de pâte à papier en fonction du temps d'ouverture des vannes.

Phase 4. Bien que des états soient indiqués dans le tableau pour cette phase il s'agit d'une phase transitoire pendant laquelle la vanne 203 est en cours de fermeture sous l'effet de l'actionneur 204. Pendant cette phase, la vanne 207 est ouverte pour injecter de l'eau pure sous haute pression, supérieure à celle de la pâte à papier dans la conduite 1 pour rincer le siège de soupape 203 et éviter des fuites dues à un coincement de fibres au moment de la fermeture.

On revient ensuite à la phase 1 et l'échantillon prélevé contenu dans la chambre 201 et la conduite 202 s'écoule dans le fractionneur.

Le préleveur décrit et illustré en figure 2 l'a été uniquement pour montrer qu'il était possible, de façon industrielle, de prélever automatiquement des échantillons de volume déterminé dans la conduite de pâte à papier 1 et les injecter dans le fractionneur 3. Tout autre moyen d'injection de volumes donnés d'échantillons de pâte à l'entrée du fractionneur pourra être imaginé par l'homme de l'art sans sortir du domaine de l'invention.

Le fractionneur 3 peut par exemple être constitué d'un tuyau en matière plastique semi-rigide d'une section de quelques dizaines à quelques centaines de mm2 et d'une longueur de l'ordre de quelques dizaines à quelques centaines de mètres, ce tuyau étant par exemple enroulé pour limiter l'encombrement

## CELLULE DE MESURE

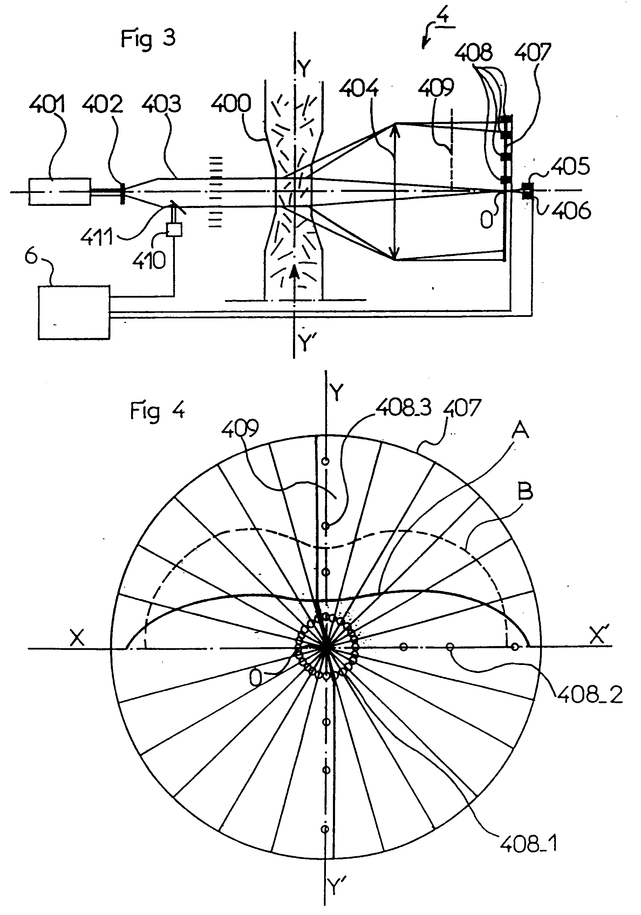

La figure 3 représente schématiquement un mode de réalisation de la cellule de mesure 4.

Le fluide sortant du fractionneur 3 circule dans une veine de mesure 400 constituée d'une portion de conduite à section réduite et à faces planes parallèles en un matériau transparent à un rayonnement électromagnétique polarisé, de préférence associé à un convergent d'entrée et un divergent de sortie. La source de rayonnement est par exemple un laser à hélium néon polarisé 401 dont le faisceau est expansé par un système optique 402 pour fournir un faisceau de rayons parallèles 403 ayant par exemple un diamètre de plusieurs millimètres qui est envoyé dans la partie de section réduite 400.

Le faisceau est collecté par une lentille 404 et, en l'absence de particules, est focalisé au point O d'un plan de mesure 407. Des phototransistors ou autres photodétecteurs 405 et 406 sont placés de part et d'autre du point O et en son proche voisinage. L'un des phototransistors, par exemple le phototransistor 405, est précédé d'un polariseur 409 assurant l'extinction du faisceau incident en l'absence de dépolarisation. Le polariseur 409 dont la direction de polarisation est orthogonale au plan de polarisation du faisceau incident, lui-même disposé à 45° de l'axe principal (YY') d'orientation des fibres dans la veine, est placé sur une moitié du plan d'analyse qui est ainsi séparé en deux demi-secteurs circulaires délimités par le diamètre YY' comme le montre la figure 4.

Quand des particules circulent dans la veine de mesure 400, le faisceau est diffracté, diffusé, et éventuellement dépolarisé. Son intensité dans diverses directions est détectée dans le plan de mesure 407 par les photodétecteurs 405 et 406 et par des photodétecteurs excentrés 408.

La figure 4 représente une vue de face du plan de mesure 407 et la disposition des photodétecteurs 408 dans ce plan. Avant de décrire cette figure plus en détail, on notera également en figure 3 que les divers photodétecteurs 405, 406, 408, ainsi qu'un détecteur supplémentaire 410 recevant une partie du faisceau incident prélevé par exemple par un miroir semi-transparent 411 sont connectés à l'automate programmable 6 pour traitement. L'automate utilisera en particulier le signal du détecteur 410 comme référence de l'intensité du faisceau laser 401. En outre, une variation de signal sur les signaux des phototransistors 405 et 406 permettra d'indiquer le début de passage des fibres dans la veine de mesure. Un étalonnage de l'appareil permettra de déterminer, en fonction du volume d'éluant qui s'est écoulé, la longueur des premières fibres arrivant, puis de connaître à tout instant la longueur des fibres traversant la veine de mesure, cet étalonnage dépendant du volume d'échantillon prélevé par le préleveur 2, des caractéristiques du fractionneur 3, et de la composition du prélèvement.

On va maintenant décrire plus en détail le fonctionnement du système de mesure en considérant la disposition des capteurs 408 dans le plan de mesure 407. Dans cette figure, les photodétecteurs sont représentés par de petits cercles. Un premier groupe de capteurs 408-1 est réparti sur un cercle centré sur la tache focale , un second groupe de capteurs 408-2 est placé sur le demi-diamètre OX' ; et un troisième groupe de capteurs 408-3 est placé selon le diamètre YY'. Le polariseur 409 s'étend sur la moitié du plan de mesure, et est limité par une frontière indiquée en traits gras, correspondant sensiblement à l'axe YY' mais s'étendant au-delà des capteurs 408-3 dans la partie supérieure de la figure et en deçà dans la partie inférieure de la figure.

Cette disposition des capteurs permet d'analyser la figure de diffraction et l'état de polarisation du faisceau ayant traversé la veine de mesure.

On a également représenté dans le demi-plan supérieur de la figure 4 la répartition de l'intensité reçue dans le plan de mesure 407 dans deux cas particuliers. La courbe A représente la répartition de l'intensité diffractée pour un ensemble d'éléments fibreux relativement rectilignes orientés préférentiellement suivant l'axe YY', c'est-à-dire l'axe de la veine de mesure. La courbe B représente la répartition de l'intensité pour des éléments fibreux dont la courbure est plus prononcée mais dont l'orientation moyenne est toujours YY'. La comparaison des signaux des capteurs 408-1 donne une information caractérisant la courbure des fibres.

En pratique, certains types d'éléments passant dans la veine de mesure provoquent une dépolarisation de la lumière (c'est par exemple le cas des fibres de cellulose). On obtient alors, comme cela est représenté, des courbes A et B, symétriques par rapport à l'axe OY (au facteur d'atténuation du polariseur 409 près). D'autres particules ne dépolariseront pas la lumière. L'intensité reçue derrière le polariseur 409 sera alors très faible. On obtiendra donc des courbes asymétriques par rapport à OY. Ainsi, les signaux fournis par les photodétecteurs 408-1 fourniront, outre une information sur la courbure, des informations sur la nature des éléments passant dans la veine de mesure.

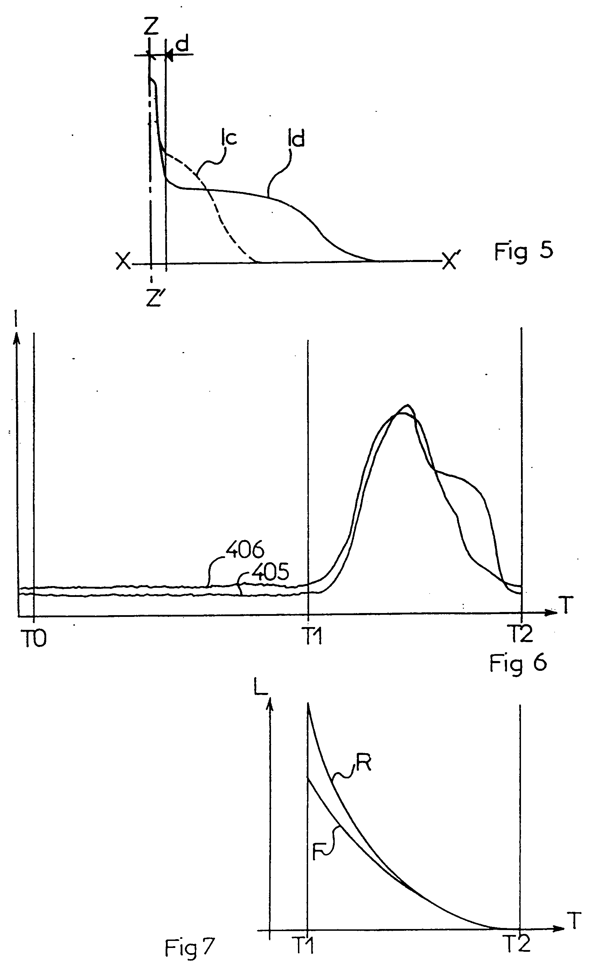

La figure 5 illustre à titre d'exemple la répartition de l'intensité du rayonnement diffracté selon la direction XX' par des particules de différents diamètres et de longueur donnée. Cette mesure est effectuée par les capteurs 408-2 se trouvant sur l'axe XX'. La courbe $I_c$ représente la répartition d'énergie diffractée pour des particules de diamètre relativement important et la courbe $I_d$ la répartition d'énergie pour des particules de diamètre plus faible. On voit que, dans les deux cas, l'intensité est relativement importante au voisinage du centre, dans la zone désignée par d, puis que l'intensité décroit plus ou moins lentement avant de chuter. Cette décroissance est d'autant plus marquée que les particules ont un diamètre plus grand. Ainsi, la répartition d'intensité selon l'axe XX' donne une indication du diamètre des fibres en cours de circulation.

Les capteurs 408-3 disposés sur l'axe YY' sont plus particulièrement destinés à caractériser les particules d'aspect plus ou moins isométrique et de petites dimensions. On rappelle que le polariseur 409, croisé par rapport à la direction de polarisation du laser source est placé devant les capteurs 408-3 le long du demi-axe OY, et non pas devant les capteurs 408-3 disposés le long du demi-axe OY'. On obtient donc une répartition d'intensité caractéristique d'une éventuelle dépolarisation et dont le résultat permet de différencier les particules minérales des fibres cellulosiques.

Bien que l'on ait indiqué ci-dessus une disposition et une utilisation particulières des capteurs, l'homme de l'art saura utiliser les signaux des divers capteurs pour en tirer d'autres informations. Dans le cas le plus général, on pourra recopier l'image sur le plan 407 dans une caméra et en analyser les diverses composantes de façon choisie.

D'autre part, alors que l'on a décrit précédemment une veine de mesure à cellule à face parallèle, on pourrait adapter une cellule cylindrique, c'est-à-dire une simple portion de tube de verre (qui n'est alors pas un capillaire). En ce cas, ce qui précède s'applique mutatis mutandis, le plan de mesure devenant une surface de mesure cylindrique.

## CYCLE DE MESURE

Ayant décrit précédemment l'organisation générale du dispositif de mesure selon l'invention et des modes de réalisation particuliers de certains de ses constituants, on va maintenant décrire une séquence de mesure, en notant qu'une telle séquence se répètera périodiquement. Ces séquences de mesure sont fixées par l'automate programmable 6.

En considérant le tableau 1, à un instant To au début de la phase 1, immédiatement après la phase 4 d'injection d'un échantillon, de l'éluant pur circule dans la veine de la cellule de mesure optique. A partir de cet instant, comme l'indique la figure 6, les capteurs 405, 406 et 408 fournissent uniquement des signaux de bruit. A partir d'un instant T1, les capteurs commencent à fournir des signaux plus intenses, ce qui indique que les premières fibres (les plus longues) arrivent dans la cellule de mesure. La figure 6 représente un exemple de la variation d'intensité reçue par les capteurs 405 et 406 dans l'intervalle de temps T1-T2 de passage de l'échantillon dans la veine de mesure. Comme on l'a vu précédemment, le capteur 406 reçoit la lumière en provenance de la source 401 ayant traversé l'ensemble du système mais sans être passé par un analyseur de polarisation. Le capteur 405 lui reçoit la lumière ayant traversé l'analyseur de polarisation 409. Ainsi, une première utilité des capteurs 405 et 406 est de donner la quantité de fibres de différentes longueurs circulant dans la veine de mesure à chaque instant.

Pour des volumes d'éluant donnés en circulation avec un débit maintenu soigneusement constant, il est possible comme l'indique la figure 7 d'étalonner le système pour faire correspondre à chaque instant dans l'intervalle de temps T1-T2 une longueur L des fibres passant dans la cellule de mesure. En figure 7, la courbe marquée R correspond à un étalonnage pour des fibres de résineux et la courbe F à un étalonnage pour des fibres de feuillus.

Les informations concernant les instants T1 et T2 étant reçues par l'automate programmable 6, celui-ci déclenche en conséquence les prises d'information à partir des divers capteurs 405, 406, 408-1, 408-2 et 408-3 décrits précédemment en relation avec la figure 4. L'automate programmable peut également choisir d'effectuer des mesures pendant l'intervalle de temps T0-T1 où, normalement, ne circule que de l'éluant pur pour détecter des niveaux de bruit qui seront soustraits des niveaux de mesure des divers capteurs.

En outre, l'automate programmable pourra commander la réinjection d'échantillons à des instants choisis, espacés d'une durée supérieure à l'intervalle de temps T1-T2 pour qu'il n'y ait pas de recouvrement dans le fractionneur 3. De plus, comme cela a été indiqué, la sortie du flux circulant dans la veine de mesure peut être dirigée vers des réservoirs montés sur un carrousel pour envoyer séquentiellement, au cours de l'intervalle de temps T1-T2, le produit dans les divers réservoirs portés par le carrousel. On dispose ainsi d'un moyen de tri des particules par catégorie de longueur qui permet aussi d'effectuer des vérifications a posteriori sur les mesures optiques précédemment effectuées ou toute autre mesure.

La présente invention vise plus particulièrement la caractérisation de la morphologie des pâtes à papier au cours des processus de fabrication sur les chaînes de production des papiers, cartons et matériaux similaires principalement constitués de particules fibreuses. La mesure de la longueur des fibres peut être utilisée, par exemple, pour le réglage des raffineurs de pâtes à papier et la mesure de la proportion des éléments organiques et minéraux pour le réglage de la composition fibreuse ainsi que le contrôle de la rétention des charges minérales dans le processus de fabrication du papier. La présente invention peut aussi être utilisée en laboratoire et pour la détermination de la morphologie de tous les types de particules, mono ou polydispersées en suspension dans un liquide. Une application de la présente invention sera de coupler le système décrit ci-avant avec la visualisation des particules en écoulement dans la suspension avant et/ou après leur classement, permettant ainsi le rapprochement des informations quantitatives recueillies dans le procédé présentement décrit d'avec les caractères spécifiques subjectifs des particules lorsqu'elles sont observées en mouvement dans la suspension en écoulement visualisée par l'image. Le dispositif décrit facilite les mesures par l'analyse d'image d'échantillons préalablement prélevés et classés en vue de mesures quantitatives réalisées par des logiciels établis.

D'autre part, alors que l'on a décrit précédemment un préleveur, un fractionneur et une cellule de mesure particuliers, l'homme de l'art pourra choisir d'autres dispositifs équivalents plus simples ou plus complexes.

## Revendications

1. Dispositif de détermination de caractéristiques de particules en suspension dans un liquide, comprenant :
   des moyens d'injection d'échantillons de particules,
   une veine de mesure optique (4) comprenant des moyens pour envoyer un rayonnement électromagnétique cohérent polarisé à travers un flux de particules en suspension et pour recueillir la lumière diffractée, éventuellement dépolarisée partiellement absorbée et diffusée dans diverses directions, et
   des moyens de calcul des valeurs des intensités et de combinaisons de ces valeurs dans les diverses directions du rayonnement optique,
   caractérisé en ce qu'il comprend, entre les moyens d'injection et la veine de mesure, un élément de fractionnement (3) qui classe les particules injectées en fonction d'un premier paramètre et les fournit à sa sortie de façon ordonnée et déterminée temporellement en fonction de ce premier paramètre.

2. Dispositif selon la revendication 1, caractérisé en ce que l'élément de fractionnement est un classeur hydrodynamique effectuant un classement privilégiant la longueur des particules, cet élément étant constitué d'un tuyau dont la section est faible par rapport à sa longueur, des quantités données d'échantillon de particules en suspension dans un liquide étant injectées à des instants déterminés dans le tuyau dans lequel un éluant circule à débit contrôlé.

3. Dispositif selon la revendication 1, caractérisé en ce qu'il comprend en outre à la sortie de la veine de mesure optique un carrousel (5) dont le fonctionnement est synchronisé avec les périodes d'injection d'échantillon de particules en suspension pour recueillir des fractions successives du fluide contenant des particules classées selon le premier paramètre.

4. Dispositif selon la revendication 1, caractérisé en ce qu'il comprend des moyens (2) pour prélever automatiquement à des instants déterminés des volumes donnés d'échantillon d'un fluide contenant des particules en suspension à partir d'une conduite (1) d'un processus industriel et pour les injecter dans ledit dispositif de classement.

5. Dispositif selon la revendication 2, caractérisé en ce qu'il comprend un automate programmable (6) assurant le séquencement entre l'injection des échantillons dans l'élément de fractionnement et les prises de mesure par les capteurs associés à la veine de mesure optique pour mesurer notamment à chaque instant correspondant à des particules de longueur données, leur quantité, leur largeur, leur courbure et la proportion entre les éléments minéraux et organiques.

6. Dispositif selon la revendication 1, caractérisé en ce que la veine de mesure est un tube transparent de section cylindrique.

**7.** Dispositif selon la revendication 2, caractérisé en ce que les moyens pour recueillir la lumière comprennent des photodétecteurs (408-1) disposés selon un cercle pour permettre une analyse sectorielle de l'intensité lumineuse diffractée et en ce que les moyens de calcul comprennent des moyens pour en déduire un facteur de forme des particules dans la veine de mesure.

**8.** Dispositif selon la revendication 1, caractérisé en ce que le rayonnement envoyé dans la veine de mesure est polarisé à 45° de l'axe d'orientation préférentielle des particules allongées dans la veine de mesure et en ce qu'une partie des photodétecteurs recueillant la lumière ayant traversé la veine de mesure est précédée d'un analyseur croisé (409).

**9.** Dispositif selon la revendication 8, caractérisé en ce qu'il comprend des moyens pour comparer les signaux de photodétecteurs disposés symétriquement par rapport audit axe d'orientation préférentielle dans la zone de lumière diffractée, les uns étant derrière ledit analyseur, les autres non.

## Claims

**1.** A device for determining the characteristics of particles in suspension in a liquid, comprising:
- injection means for injecting samples of particles,
- an optical measurement cell (4) including means for sending a polarized coherent electromagnetic radiation through a flow of particles in suspension and for collecting the diffracted, possibly depolarized light, partially absorbed and diffused in various directions, and
- means for calculating the values of the intensities and combinations of those values in the various directions of the optical radiation,
    characterized in that it further includes between said injection means and said measurement cell,
- a fractionator (3) which sorts the injected particles as a function of a first parameter and supplies them at its output in a time sequenced and determined mode as a function of said first parameter.

**2.** A device according to claim 1, characterized in that the fractionator is a hydrodynamic sorting device, sorting in priority the particles as a function of their length, said fractionator being made of a tube, the section of which is low with respect to its length, determined quantities of sample of particles in suspension in a liquid being injected at determined times into the tube through which an eluant flows at a controlled flow rate.

**3.** A device according to claim 1, characterized in that it further comprises at the output of the optical measurement cell a turntable (5), the operation of which is synchronized with the periods during which the samples of particle in suspension are injected for collecting successive fractions of the fluid containing particles sorted according to said first parameter.

**4.** A device according to claim 1, characterized in that it comprises means (2) for automatically drawing at determined times given sample volumes of a liquid containing particles in suspension from a tube (1) of an industrial line and injecting them into said fractionator.

**5.** A device according to claim 2, characterized in that it comprises a programmable controller (6) sequencing injection of the samples into the fractionator and measurements by detectors associated with the optical measurement cell for more particularly measuring at each moment corresponding to particles of a given length, their quantity, width, curvature and the ratio between the mineral and organic elements.

**6.** A device according to claim 1, characterized in that said measurement cell is a transparent tube with a cylindrical section.

**7.** A device according to claim 2, characterized in that said means for collecting light comprise photodetectors (408-1) arranged in a circle for allowing the diffracted light intensity to be sectorially analyzed and wherein said calculating means comprise means for deducing a shape factor of particles in the measurement cell.

**8.** A device according to claim 1, characterized in that the light radiation sent into the measurement cell is polarized at an angle of 45° with respect to the direction of the preferential axis of the particles longitudinally positioned in the measurement cell, and wherein a portion of said photodetectors collecting the light which has passed through the measurement cell is preceded by a crossed polarization analyzer (409).

9. A device according to claim 8, characterized in that it comprises means for comparing the signals of the photodetectors symmetrically arranged with respect to said preferential axis in the diffracted light region, some of the photodetectors being placed behind said analyzer, and other not.

**Patentansprüche**

1. Vorrichtung zum Bestimmen der Eigenschaften von in einer Flüssigkeit suspendierten Teilchen mit
   - einer Einrichtung zum Injizieren von Proben aus Teilchen,
   - einer optischen Meßzelle (49) mit einer Einrichtung zum Abgeben einer kohärenten polarisierten elektromagnetischen Strahlung quer durch einen suspendierten Teilchenfluß und zum Erfassen des abgelenkten, gegebenenfalls depolarisierten, teilweise absorbierten und in verschiedenen Richtungen gestreuten Lichtes und
   - einer Recheneinrichtung zur Berechnung der Intensitatswerte und Kombinationen dieser Werte der optischen Strahlung in verschiedenen Richtungen,

   dadurch gekennzeichnet, daß sie zwischen der Injektionseinrichtung und der Meßzelle ein Fraktionierungselement (3) aufweist, das die injizierten Teilchen als Funktion eines ersten Parameters klassifiziert und diese an seinem Ausgang mit geordneter und zeitweise bestimmter Art als Funktion dieses ersten Parameters abgibt.

2. Vorrichtung nach Anspruch 1, dadurch gekennzeichnet, daß das Fraktionierungselement ein hydrodynamischer Sortierer ist, der bevorzugt eine Klassifizierung nach der Lange der Teilchen vornimmt, und daß dieses Element aus einem Rohr besteht, dessen Querschnitt klein gegenüber seiner Länge ist, wobei gegebene Mengen von Proben aus in einer Flüssigkeit suspendierten Teilchen zu bestimmten Zeitpunkten in das Rohr injiziert werden, in dem ein Elutionsmittel mit kontrolliertem Durchfluß zirkuliert.

3. Vorrichtung nach Anspruch 1, dadurch gekennzeichnet, daß sie des weiteren am Ausgang der optischen Meßzelle einen Drehtisch (5) aufweist, dessen Funktion mit den Injektionsperioden der Proben aus Teilchen in der Suspension synchronisiert ist, um die aufeinanderfolgenden Fraktionen der die nach dem ersten Parameter klassifizierten Teilchen enthaltenden Flüssigkeit zu sammeln.

4. Vorrichtung nach Anspruch 1, dadurch gekennzeichnet, daß sie eine Einrichtung (2) zum automatischen Entnehmen von vorgegebenen Probenvolumina einer Flüssigkeit mit suspendierten Teilchen zu bestimmten Zeitpunkten aus einer Leitung eines industriellen Prozesses und zum Injizieren dieser Volumina in die Klassifizierungseinrichtung aufweist.

5. Vorrichtung nach Anspruch 2, dadurch gekennzeichnet, daß sie eine programmierbare Steuerung (6) aufweist, die die Abfolge zwischen der Injektion von Proben in das Fraktionierungselement und den Erfassungen der Meßwerte durch die der optischen Meßzelle zugeordneten Fühler sicherstellt, um insbesondere zu jedem Zeitpunkt unter den Teilchen gegebener Länge deren Menge, Breite, Krümmung und das Verhältnis zwischen mineralischen und organischen Elementen zu messen.

6. Vorrichtung nach Anspruch 1, dadurch gekennzeichnet, daß die Meßzelle ein transparentes Rohr mit zylindrischem Querschnitt ist.

7. Vorrichtung nach Anspruch 2, dadurch gekennzeichnet, daß die Einrichtung zum Sammeln des Lichtes Fotodetektoren (408-1) aufweist, die längs eines Kreises angeordnet sind, um eine sektorielle Analyse der Intensität des abgelenkten Lichtes zu ermöglichen, und daß die Recheneinrichtung Mittel aufweist, um daraus einen Formfaktor der Teilchen in der Meßzelle abzuleiten.

8. Vorrichtung nach Anspruch 1, dadurch gekennzeichnet, daß die in die Meßzelle gesendete Strahlung unter einem Winkel von 45° in bezug auf die bevorzugte Orientierungsachse der länglichen Teilchen in der Meßzelle polarisiert ist, und daß ein Teil der Fotodetektoren, die das die Meßzelle durchquerte Licht sammeln, einen vorgeschalteten Kreuzanalysator (409) aufweisen.

9. Vorrichtung nach Anspruch 8, dadurch gekennzeichnet, daß sie eine Einrichtung zum Vergleich der Signale der Fotodetektoren aufweist, die in bezug zu der genannten bevorzugten Orientierungsachse in der Zone des abgelenkten Lichtes angeordnet sind, wobei die einen hinter dem besagten Analysator angeordnet sind und die anderen nicht.

Fig 1

## Fig 2

Fig 3

Fig 4

Fig 5

Fig 6

Fig 7